# EUROPEAN PATENT SPECIFICATION

(11) **EP 1 296 951 B1**
(45) Date of publication and mention of the grant of the patent: **06.10.2010**
(21) Application number: 01939267.9
(22) Date of filing: 22.05.2001
(51) Int. Cl.: C07D 213/61, A61K 31/444, A61P 29/00

(54) **5-CHLORO-3-(4-METHANESULFONYLPHENYL)-6'-METHYL- [2,3'] BIPYRIDINYL IN PURE CRYSTALLINE FORM AND PROCESS FOR SYNTHESIS**
KRISTALLINE FORM VON 5-CHLORO-3-(4-METHANESULFONYLPHENYL)-6'-METHYL- [2,3'] BIPYRIDINYL UND HERSTELLUNGSVERFAHREN HIERFÜR
5-CHLORO-3-(4-METHANESULFONYLPHENYL)-6'-METHYL- [2,3'] BIPYRIDINYL SOUS FORME CRISTALLINE PURE ET PROCEDE DE SYNTHESE

(30) Priority: 26.05.2000 US 208017 P
(43) Date of publication of application: 02.04.2003
(73) Proprietor: Merck Sharp & Dohme Corp., Rahway, NJ 07065 (US)
(72) Inventor: CROCKER, Louis, S., Rahway, NJ 07065-0907 (US); DAVIES, Ian, W., Rahway, NJ 07065-0907 (US); OSIFCHIN, Richard, G., Rahway, NJ 07065-0907 (US); KOTLIAR, Andrew, Rahway, NJ 07065-0907 (US)
(74) Representative: Horgan, James Michael Frederic
(86) International application number: PCT/US2001/016566
(87) International publication number: WO 2001/092230

(56) References cited:
- WO-A-01/37833
- WO-A-98/03484
- WO-A-99/55830
- FRIESEN R W ET AL: "2-pyridinyl-3-(4-methylsulfonyl)phenylpyr idines: selective and orally active cyclooxygenase-2 inhibitors" BIOORGANIC & MEDICINAL CHEMISTRY LETTERS, OXFORD, GB, vol. 8, no. 19, 6 October 1998 (1998-10-06), pages 2777-2782, XP004139619 ISSN: 0960-894X cited in the application

## Description

### BACKGROUND OF THE INVENTION

The present invention relates to the Form V polymorph of Compound A having the chemical structure shown below: 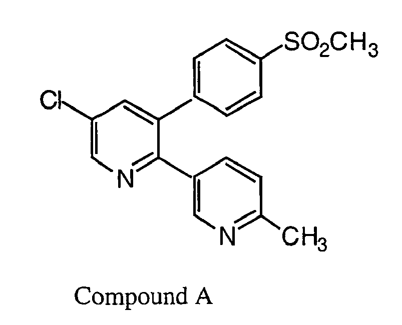 as well as a process for synthesizing the Form V polymorph.

Compound A exists in five polymorphic forms (Forms I-V), an amorphous form and two hydrated forms. The compound is a potent and selective cyclooxygenase-2 (COX-2) inhibitor, useful primarily in the treatment of inflammation, pain and fever as well as other COX-2 mediated diseases, such as described in PCT Publication Nos. WO96/10012 and WO96/16934. Compound A is described in U.S. Patent No. 5,861,419 granted on January 19, 1999 (Example 23), which is hereby incorporated by reference in its entirety. A process for making Compound A is described in U.S. Patent No. 6,040,319 granted on March 21, 2000, which is hereby incorporated by reference in its entirety. The present invention unexpectedly provides for a novel, robust process for making a Form V polymorph of compound A from any one of Forms I, II, III or IV or any mixture of polymorphs of compound A.

### SUMMARY OF THE INVENTION

This invention encompasses the Form V polymorph of structural formula A: 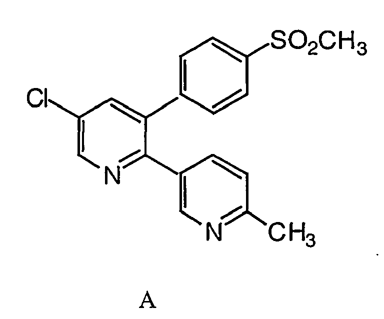 which is useful in the treatment of cyclooxygenase-2 mediated diseases.

The invention encompasses certain pharmaceutical compositions for the treatment of cyclooxygenase-2 mediated diseases comprising the Form V polymorph of Compound A. The invention also encompasses a process for synthesizing the Form V polymorph of Compound A comprising: combining polymorph I, II, III or IV of Compound A with isopropyl acetate; heating to an elevated temperature less than about 75°C; and cooling to a low temperature to produce the Form V polymorph.

### BRIEF DESCRIPTION OF THE DRAWINGS

The invention is described in connection with the appended drawings in which:
Fig. 1 is the X-ray powder diffraction (XRPD) pattern of Form V;
Fig. 2 is the XRPD pattern of Form I;
Fig. 3 is the XRPD pattern of Form II;
Fig. 4 is the XRPD pattern of Form III;
Fig. 5 is the XRPD pattern of Form IV;
Fig. 6 is the XRPD pattern of the hemihydrate; and
Fig. 7 is the XRPD pattern of the sesquihydrate.

### DETAILED DESCRIPTION

This invention encompasses the Form V polymorph of structural formula A: 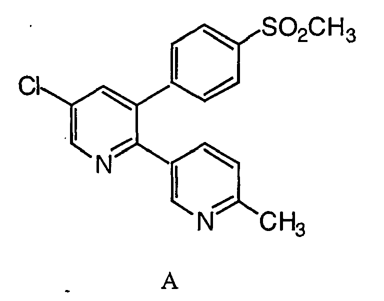 having the following physical characteristics: DSC extrapolated onset melting temperature of 133.9°C, DSC peak melting temperature of 134.5°C, and x-ray powder diffraction peak positions, Cu K alpha, of: 13.7, 7.2, 6.9, 6.7, 5.8, 5.7, 5.0, 4.9, 4.8, 4.7, 4.5, 4.2, 4.0, 3.9, 3.8, 3.7, 3.6, 3.4, 3.3, 3.1, 3.0, 2.9 and 2.8 angstroms.

An embodiment of the invention is the Form V polymorph of Compound A characterized as having an x-ray powder diffraction pattern peak position, Cu K alpha, at about 13.7 angstroms. Within this embodiment of the invention is the Form V polymorph of Compound A further characterized as having at least one x-ray powder diffraction pattern peak position, Cu K alpha, at about: 7.2, 6.9, 6.7, 5.8, 5.7, 5.0, 4.9, 4.8, 4.7, 4.5, 4.2, 4.0, 3.9, 3.8, 3.7, 3.6, 3.4, 3.3, 3.1, 3.0, 2.9 or 2.8 angstroms.

An embodiment of the invention is the Form V polymorph of Compound A characterized as having a DSC extrapolated onset melting temperature of about 133.9°C.

An embodiment of the invention is the Form V polymorph of Compound A characterized as having a DSC peak melting temperature of about 134.5°C.

An embodiment of the invention is the Form V polymorph of Compound A having the aforesaid characteristics in substantially pure form.

The invention also encompasses a pharmaceutical composition comprising a non-toxic therapeutically effective amount of a Form V polymorph of Compound A and a pharmaceutically acceptable carrier.

An embodiment of the invention encompasses polymorphic form V of the compound of formula A for use in a method of treating an inflammatory disease susceptible to treatment with an non-steroidal anti-inflammatory agent comprising administering to a patient in need of such treatment a non-toxic therapeutically effective amount of a Form V polymorph of Compound A.

Another embodiment of the invention encompasses polymorphic form V of the compound of formula A for use in a method of treating a cyclooxygenase mediated disease advantageously treated by an active agent that selectively inhibits cyclooxygenase-2 in preference to cyclooxygenase-1 comprising administering to a patient in need of such treatment a non-toxic therapeutically effective amount of a Form V polymorph of Compound A.

Another embodiment of the invention encompasses polymorphic form V of the compound of formula A for use in a method for treating an illness selected from the group consisting of:
(a) rheumatic fever,
(b) symptoms associated with influenza or other viral infections, common cold,
(c) low back and neck pain,
(d) dysmenorrhea,
(e) headache,
(f) toothache,
(g) sprains and strains,
(h) myositis,
(i) neuralgia,
(j) synovitis,
(k) arthritis, including rheumatoid arthritis, degenerative joint diseases (osteoarthritis), gout and ankylosing spondylitis,
(l) bursitis,
(m) burns,
(n) injuries, and
(o) following surgical and dental procedures,
comprising administering to a patient in need of such treatment a non-toxic therapeutically effective amount of a Form V polymorph of Compound A.

This invention also encompasses a novel process for making a Form V polymorph of structural formula A comprising: combining polymorph I, II, III or IV of Compound A with isopropyl acetate; heating to an elevated temperature less than about 75°C; and cooling to a low temperature to produce the Form V polymorph.

For purposes of this Specification, the term 'elevated temperature' means any temperature above room temperature but less than about 75°C, as high as about 35-70°C, preferably about 50-65°C. Room temperature is about 20°C. The term 'low temperature' means any temperature below the 'elevated temperature', as low as about 0-30°C, preferably as low as about 10-20°C.

Polymorphic forms of Compound A, for purposes of this invention, are identified as Form I (onset of melting m.p. 135.7 ± 0.2°C, peak m.p. 137.0 ± 0.2°C), Form II (onset of melting m.p 129.6°C, peak m.p. 131.5°C), Form III (onset of melting m.p. 133.2°C, peak m.p. 134.4°C), Form IV (onset of melting m.p. 133.72 ± 0.04°C, peak m.p. 134.5 ± 0.1°C) and Form V (onset of melting, m.p. 133.9°C, peak m.p. 134.5°C). Forms I through V are anhydrous.

An embodiment of the invention encompasses the process for making the Form V polymorph of Compound A which further comprises isolating the Form V polymorph. A subset of this embodiment encompasses isolating the Form V polymorph by filtration.

An embodiment of the invention is the process for making a Form V polymorph of Compound A wherein the elevated temperature is about 40-75°C. Another embodiment of the invention is the process for making a Form V polymorph of Compound A wherein the elevated temperature is about 50-65°C.

An embodiment of the invention is the process for making a Form V polymorph of Compound A wherein the low temperature is about 0-30°C. Another embodiment of the invention is the process for making a Form V polymorph of Compound A wherein the low temperature is about 10-20°C.

Another embodiment of the invention is the process for making a Form V polymorph of Compound A wherein the elevated temperature is about 50-65°C and the low temperature is about 10-20°C.

The invention will now be illustrated by the following non-limiting examples:

### PREPARATIVE EXAMPLE A

The starting material Compound A was made in accordance with U. S. Pat. No. 6,040,319.

### PREPARATIVE EXAMPLE B

### FORM II

Form II was obtained by crystallizing Compound A obtained in accordance with Preparative Example A from ethyl acetate. Differential Scanning Calorimetry showed an extrapolated onset of melting at about 130°C, and a peak melting point of about 131°C.

### PREPARATIVE EXAMPLE C

### FORM IV

Form IV was prepared by mixing Preparative Example A (550.0g, 1.54 mol) and toluene (4.0 L) and heating the mixture to 32.6°C to cause dissolution. The solution was cooled to 16.5°C and Form IV crystallized. The mixture was then cooled to 0°C over 1 hr. n-Heptane (7.0L) was added over 2 hr and the mixture was filtered. The cake was washed with 3:1 n-heptane/toluene (3.0L) and dried to give the product (521.0 g) as a granular solid.

### PREPARATIVE EXAMPLE D

### HEMIHYDRATE

A solution of Preparative Example A (65 g) in 1 L of water wet toluene was heated to 60°C and then cooled to ambient temperature. The hemihydrate form crystallized and was isolated by filtration. The solids were dried at ambient temperature under vacuum to give ∼ 30 g of a colorless crystals.

### PREPARATIVE EXAMPLE E

### FORM III

The hemihydrate of Preparative Example D was heated to 90°C in a vacuum oven for 12 hours and cooled down in the vacuum oven to give the Form III polymorph.

### PREPARATIVE EXAMPLE G

### AMORPHOUS

The amorphous form of compound A was obtained by heating the Form IV from Preparative Example C to above its melting temperature (above about 135°C) under nitrogen, followed by quench cooling to room temperature under a dry atmosphere.

### PREPARATIVE EXAMPLE H

### MIXTURE OF POLYMORPHS

Compound 1 was synthesized in accordance with Preparative Example 1 of U. S. Pat. No. 6,040,319. Compound 2 was synthesized in accordance with Example 1 of U. S. Pat. No. 6,040,319.

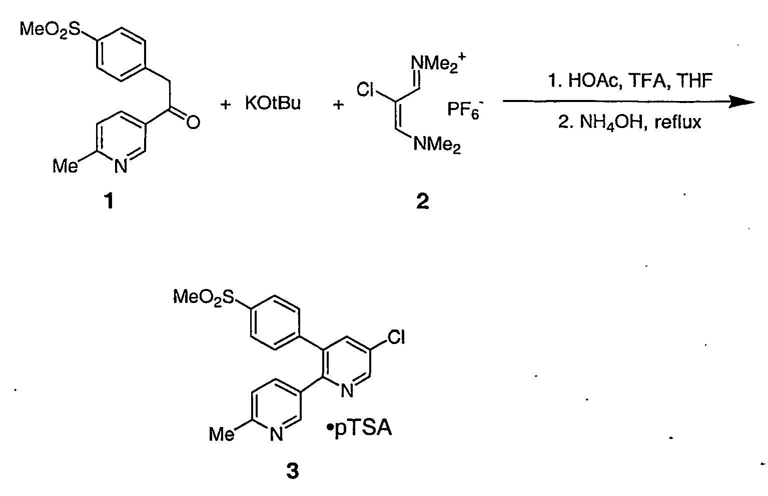

To a slurry of Compound 1 (1.10 kg) in tetrahydrofuran (THF) (2.5 L) at 0 °C was added potassium tert-butoxide (2.47 L). The resulting mixture was transferred to a slurry of Compound 2 (1.19 kg) in THF at ambient temperature. The slurry was transferred to a solution of acetic acid (1.5 L) and trifluoroacetic acid (TFA) (0.23 L) in THF. Concentrated ammonium hydroxide (1.50 L) was added and the mixture to reflux. The reaction mixture was cooled and the phases were cut. The THF layer was concentrated and toluene was added. The toluene layer was washed with aqueous sodium hydroxide followed by water and then concentrated to ∼ 6 L. Acetone was added and a solution of p-toluenesulfonic acid (pTSA) (0. 73 kg) in acetone was added and batch was filtered. The filter cake was washed with toluene/acetone and the solid dried in vacuo to give 1.80 kg of Compound 3 in ∼ 90 % isolated yield as an off white solid.

To a mixture of toluene, water and Compound 3 (1.80 kg) was added aqueous ammonia (1 equiv.). The phases were cut and the toluene layer was washed with water. The mixture was filtered through SOLKAFLOC and the filtrate was concentrated to a saturated solution and then cooled to ambient temperature and n-heptane was added. The solid was isolated by filtration, washed with toluene/n-heptane and then dried in vacuo to give Preparative Example H as an off-white solid.

### EXAMPLE 1

### FORM V OF 5-CHLORO-3-(4-METHANESULFONYLPHENYL)-6'-METHYL-[2,3']BIPYRIDINYL

A mixture of Preparative Example H and isopropyl acetate (IPAC) was heated at 55 °C. The suspension was cooled to ambient temperature and the solids were isolated by filtration. The solids were washed with IPAC and dried in vacuo to give the Form V polymorph (1.1 kg) as a colorless solid in ∼ 87 % yield.
¹H NMR (400 MHz CDCl₃) δ 8.69 (d, 1H, *J*=2.3 Hz), 8.36 (3, 1H, *J*=2.2 Hz), 7.88 (d, 2H, *J*=8.4 Hz), 7.72 (d, 1H, *J*=2.3 Hz), 7.54 (dd, 1H, *J₁*=8.0 Hz, *J₂*=2.3 Hz), 7.38 (d, 2H, *J*=8.5 Hz), 7.07 (d, 1H, *J*=8.0 Hz), 3.06 (s, 3H), 2.51 (s, 3H); 13C NMR (100 MHz CDCl₃) δ 158.4, 152.2, 149.7, 148.3, 143.7, 140.1, 137.9, 137.2, 135.18. 131.1, 130.0, 130.3, 127.8, 122.7, 44.4, 24.1.

### CHARACTERIZATION OF POLYMORPHS

The polymorphic forms of compound A were characterized using the following procedures.

### X-Ray Powder Diffraction Pattern Analysis

The X-ray patterns were collected using a Philips APD powder diffractometer utilizing copper K-alpha radiations. Table 1 below lists the XRPD peak locations for Forms I, II, III, IV and V as well as the hemihydrate and sesquihydrate forms. The peak positions are expressed in angstroms in Table 1.

**TABLE 1**

| X-ray Powder Diffraction D-spacing for Crystalline Phases in Reflections (angstroms) | | | | | | |
|---|---|---|---|---|---|---|
| Form I | Form II | Form III | Form IV | Form V | Hemi-hydrate | Sesqui-hydrate |
| 12.6 | 16.1 | 10.8 | 10.4 | 13.7 | 10.9 | 12.7 |
| 9.1 | 9.4 | 8.2 | 5.9 | 7.2 | 10.6 | 10.2 |
| 7.5 | 8.3 | 6.9 | 5.4 | 6.9 | 6.2 | 8.0 |
| 7.2 | 6.8 | 6.4 | 5.2 | 6.7 | 5.8 | 7.7 |
| 6.8 | 5.3 | 6.2 | 5.0 | 5.8 | 5.6 | 7.5 |
| 5.7 | 5.2 | 5.7 | 4.7 | 5.7 | 5.5 | 6.3 |
| 5.4 | 5.1 | 5.4 | 4.6 | 5.0 | 5.3 | 6.0 |
| 4.9 | 4.8 | 5.0 | 4.1 | 4.9 | 5.0 | 5.8 |
| 4.6 | 4.5 | 4.6 | 4.0 | 4.8 | 4.6 | 5.4 |
| 4.4 | 4.3 | 4.5 | 3.9 | 4.7 | 4.4 | 5.1 |
| 4.2 | 4.1 | 4.1 | 3.8 | 4.5 | 4.2 | 4.8 |
| 4.1 | 3.9 | 3.9 | 3.6 | 4.2 | 4.1 | 4.5 |
| 3.9 | 3.8 | 3.8 | 3.3 | 4.0 | 4.0 | 4.2 |
| 3.8 | 3.6 | 3.7 | 3.1 | 3.9 | 3.8 | 4.1 |
| 3.7 | 3.5 | 3.5 | 3.0 | 3.8 | 3.6 | 4.0 |
| 3.4 | 3.4 | 3.3 | | 3.7 | 3.4 | 3.9 |
| 3.1 | 3.2 | 3.2 | | 3.6 | 3.2 | 3.7 |
| | 3.0 | 3.1 | | 3.4 | 3.1 | 3.5 |
| | | 2.8 | | 3.3 | | 3.4 |
| | | | | 3.1 | | 3.3 |
| | | | | 3.0 | | 3.1 |
| | | | | 2.9 | | |
| | | | | 2.8 | | |

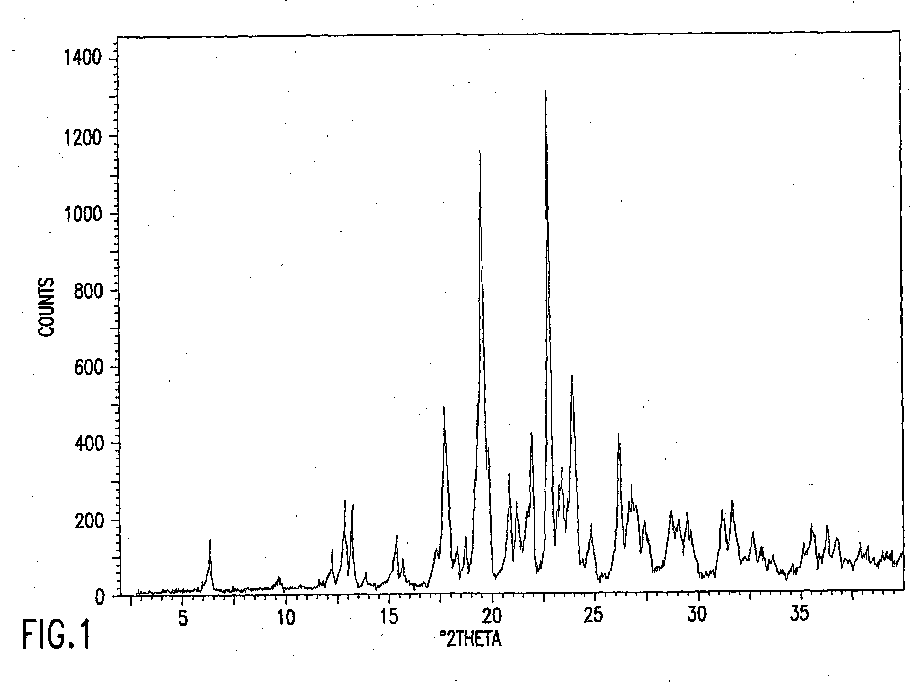

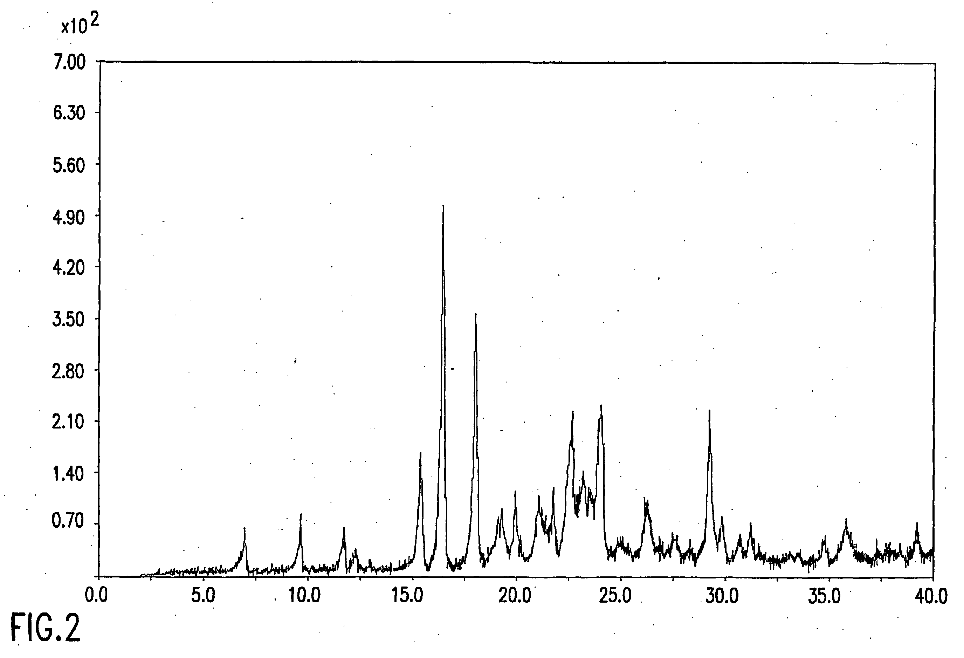

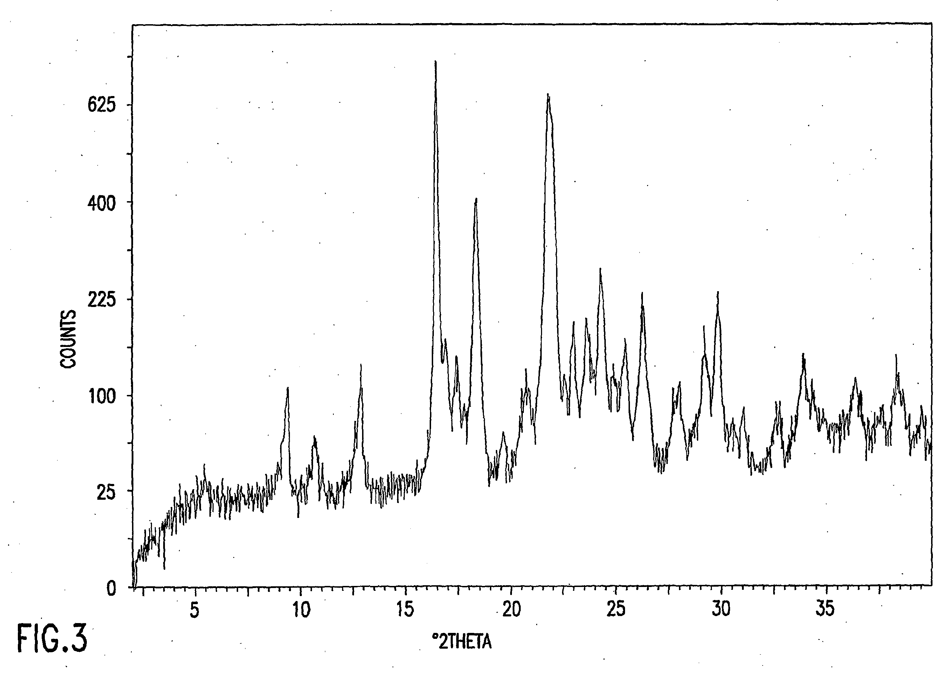

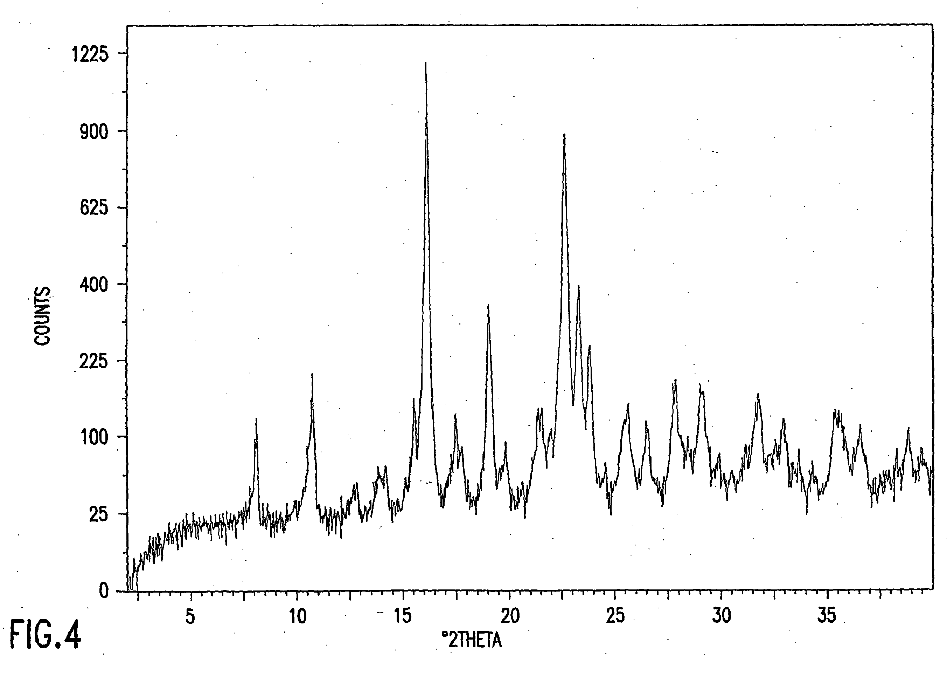

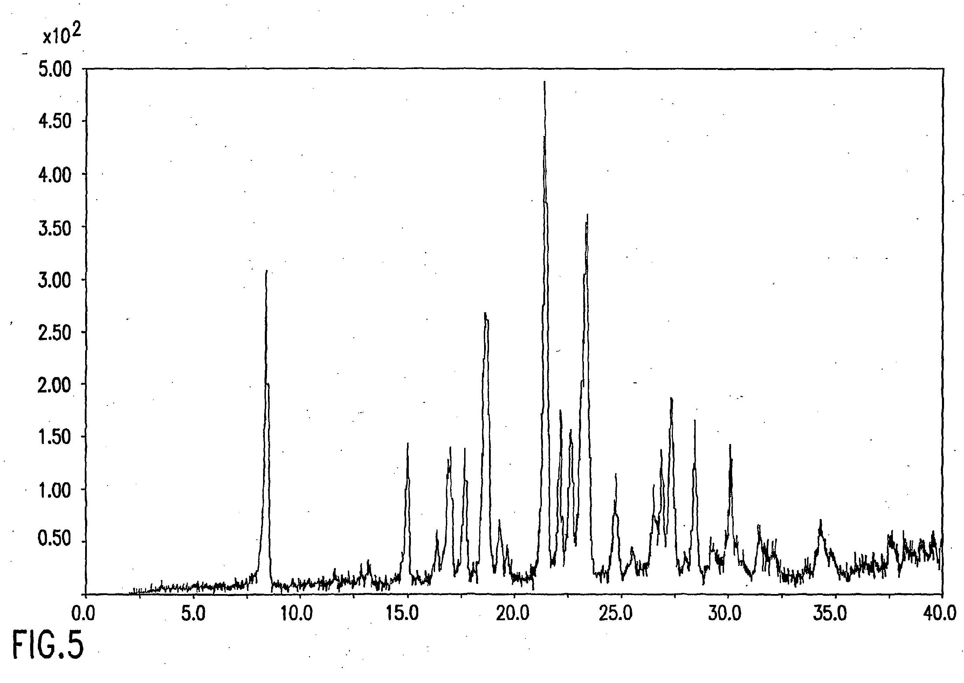

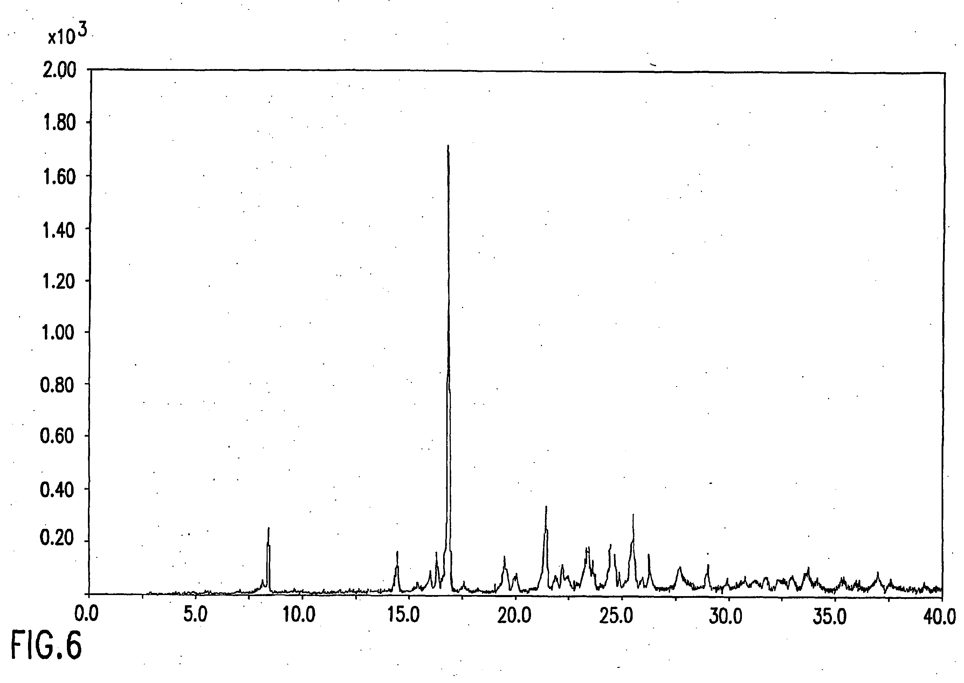

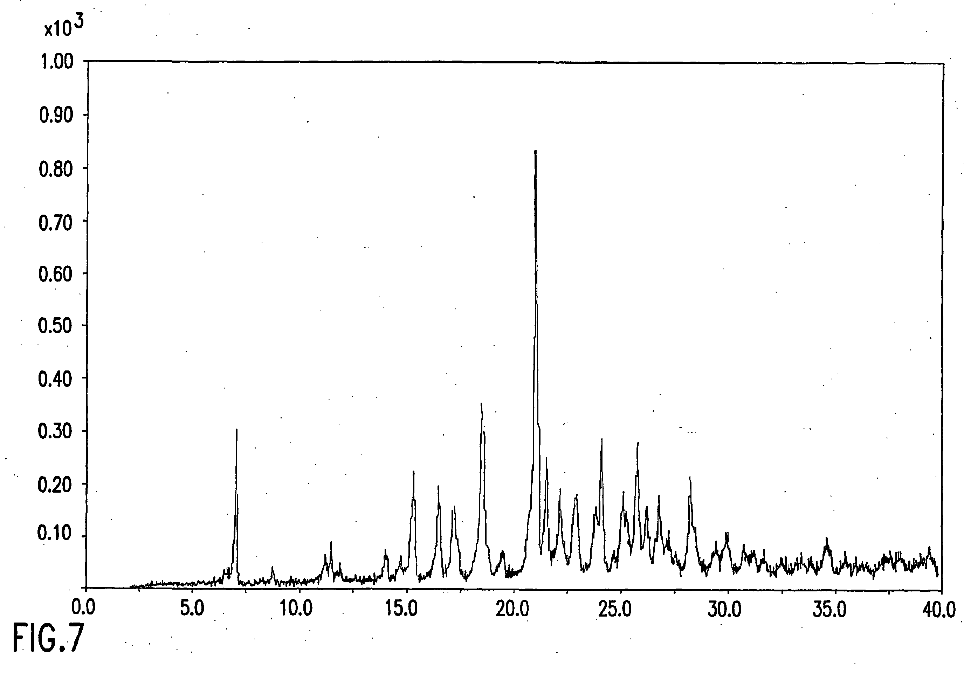

The XPRD pattern for Form V is shown in Figure 1. XRPD patterns for Forms I-IV are shown in Figures 2-5. XRPD patterns for the two hydrate forms are shown in Figures 6 and 7. The peak positions are expressed in degrees (2 theta) in the plots.

### Differential Scanning Calorimetry (DSC)

DSC was carried out using a TA Instruments DSC 2910 instrument at a heating rate of 1°C/min under a nitrogen atmosphere in an open pan. The extrapolated onset temperatures, Tₒ, and enthalpy of fusion, AH, observed for the melting endotherms are shown in Table 2 for Forms I, II, III, IV and V.

**TABLE 2**

| Extrapolated melting temperature onset, Tₒ, and Enthalpy of Fusion obtained by DSC at 1°C/min in an open pan under nitrogen | | |
|---|---|---|
| Polymorphic form | Tₒ(°C) | Enthalpy of fusion, J/g |
| Form I | 135.7 ± 0.2 | 72.9 ± 2.0 |
| Form II | 129.6 | |
| Form III | 133.2 | |
| Form IV | 133.72 ± 0.04 | 76.9 ± 1.4 |
| Form V | 133.9 | 84.8 |

## Claims

1. A polymorphic form of the compound of formula A: 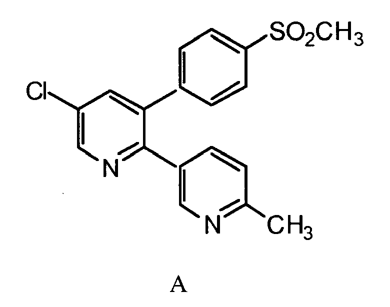 which is designated Form V, **characterized** as having an x-ray powder diffraction pattern peak position, Cu K alpha, at 13.7 angstroms.

2. The polymorphic form according to Claim 1 further **characterized** as having at least one x-ray powder diffraction pattern peak position, Cu K alpha, at: 7.2, 6.9, 6.7, 5.8, 5.7, 5.0, 4.9, 4.8, 4.7, 4.5, 4.2, 4.0, 3.9, 3.8, 3.7, 3.6, 3.4, 3.3, 3.1, 3.0, 2.9 or 2.8 angstroms.

3. The polymorphic form according to Claim 1 **characterized** as having a DSC extrapolated onset melting temperature of 133.9°C.

4. The polymorphic form according to Claim 1 **characterized** as having a DSC peak melting temperature of 134.5°C.

5. The polymorphic form according to Claim 1 in substantially pure form.

6. A pharmaceutical composition comprising a non-toxic therapeutically effective amount of a polymorphic form according to Claim 1 and a pharmaceutically acceptable carrier.

7. A polymorphic form according to any one of claims 1 to 5 for use in a method of treatment of the human or animal body by therapy.

8. Use of a polymorphic form according to any one of claims 1 to 5 for the manufacture of a medicament for treating an inflammatory disease and/or treating a cyclooxygenase mediated disease.

9. A polymorphic form according to any one of claims 1 to 5 for use in treating an illness selected from:
(a) rheumatic fever,
(b) symptoms associated with influenza or other viral infections, common cold,
(c) low back and neck pain,
(d) dysmenorrhea,
(e) headache,
(f) toothache,
(g) sprains and strains,
(h) myositis,
(i) neuralgia,
(j) synovitis,
(k) arthritis, including rheumatoid arthritis, degenerative joint diseases (osteoarthritis), gout and ankylosing spondylitis,
(l) bursitis,
(m) bums,
(n) injuries, and
(o) following surgical and dental procedures.

10. A process for making a Form V polymorph of structural formula A: 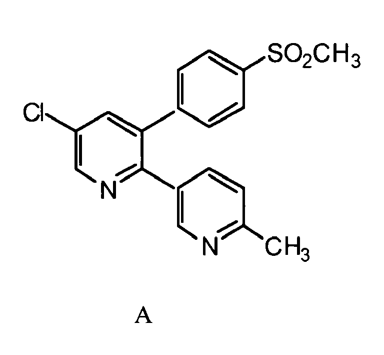 as defined in claim 1, comprising:
combining polymorph I, II, III or IV or any mixture of polymorphs of Compound A with isopropyl acetate;
heating to an elevated temperature less than about 75°C; and
cooling to a low temperature to produce the Form V polymorph;
wherein said polymorphs I, II, III and IV are **characterised by** x-ray powder diffraction pattern peak positions, Cu K alpha, at 12.6, 16.1, 10.8 and 10.4 angstroms, respectively.

11. The process according to Claim 10 further comprising isolating the Form V polymorph.

12. The process according to Claim 11 wherein isolating the Form V polymorph is by filtration.

13. The process according to Claim 10 wherein the elevated temperature is from 35-70°C.

14. The process according to Claim 13 wherein the elevated temperature is from 50-65°C.

15. The process according to Claim 10 wherein the low temperature is from 0-30°C.

16. The process according to Claim 15 wherein the low temperature is from 10-20°C.

17. The process according to Claim 10 wherein the elevated temperature is from 50-65°C and the low temperature is about 10-20°C.

## Patentansprüche

1. Eine polymorphe Form der Verbindung der Formel A: 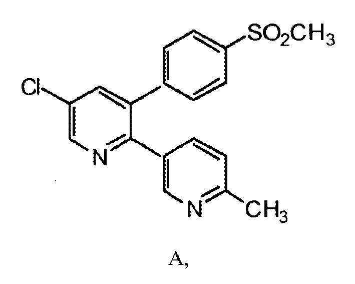 die als Form V bezeichnet wird, **dadurch gekennzeichnet, dass** sie eine Cu-K-alpha-Röntgenpulverbeugungsdiagramm-Peakposition bei 13,7 Angström besitzt.

2. Die polymorphe Form gemäß Anspruch 1, die ferner **dadurch gekennzeichnet ist, dass** sie wenigstens eine Cu-K-alpha-Röntgenpulverbeugungsdiagramm-Peakposition besitzt bei: 7,2, 6,9, 6,7, 5,8, 5,7, 5,0, 4,9, 4,8, 4,7, 4,5, 4,2, 4,0, 3,9, 3,8, 3,7, 3,6, 3,4, 3,3, 3,1, 3,0, 2,9 oder 2,8 Angström.

3. Die polymorphe Form gemäß Anspruch 1, **dadurch gekennzeichnet, dass** sie einen extrapolierten Schmelztemperaturbeginn im DSC von 133,9°C besitzt.

4. Die polymorphe Form gemäß Anspruch 1, **dadurch gekennzeichnet, dass** sie einen Schmelztemperaturspitzenwert im DSC von 134,5°C besitzt.

5. Die polymorphe Form gemäß Anspruch 1 in im Wesentlichen reiner Form.

6. Eine pharmazeutische Zusammensetzung, die eine nichttoxische therapeutisch wirksame Menge einer polymorphen Form gemäß Anspruch 1 und einen pharmazeutisch annehmbaren Träger umfasst.

7. Eine polymorphe Form gemäß einem der Ansprüche 1 bis 5 zur Verwendung bei einem Verfahren zur Behandlung des Menschen- oder Tierkörpers durch Therapie.

8. Verwendung einer polymorphen Form gemäß einem der Ansprüche 1 bis 5 zur Herstellung eines Medikaments zur Behandlung einer Entzündungserkrankung und/oder zur Behandlung einer durch Cyclooxygenase vermittelten Erkrankung.

9. Eine polymorphe form gemäß einem der Ansprüche 1 bis 5 zur Verwendung bei der Behandlung einer Krankheit, ausgewählt aus:
(a) rheumatischem Fieber,
(b) Symptomen, die mit Influenz oder anderen Virusinfektionen verbunden sind, gewöhnlicher Erkältung,
(c) Schmerzen im unteren Rücken und Nacken,
(d) Dysmenorrhö,
(e) Kopfschmerzen,
(f) Zahnschmerzen,
(g) Verstauchungen und Verrenkungen,
(h) Myositis,
(i) Neuralgie,
(j) Synovitis,
(k) Arthritis, einschließlich rheumatoider Arthritis, degenerativer Gelenkerkrankungen (Osteoarthritis), Gicht und Spondylitis ankylosans,
(l) Bursitis,
(m) Verbrennungen,
(n) Verletzungen und
(o) nach operativen und dentalen Eingriffen.

10. Ein Verfahren zur Herstellung eines Form-V-Polymorphen der Strukturformel A: 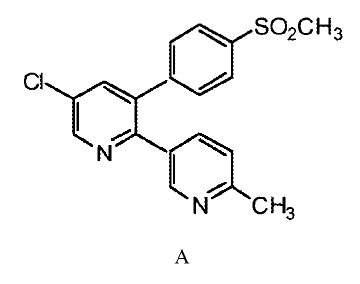 wie in Anspruch 1 definiert, umfassend:
Vereinen von Polymorph I, II, III oder IV oder einer beliebigen Mischung von Polymorphen der Verbindung A mit Isopropylacetat,
Erwärmen auf eine erhöhte Temperatur von weniger als etwa 75°C und
Abkühlen auf eine niedrige Temperatur, um das Form-V-Polymorph zu erzeugen,
wobei die Polymorphe I, II, III und IV **gekennzeichnet sind durch** Cu-K-alpha-Röntgenpulverbeugungsdiagramm-Peakpositionen bei 12,6, 16,1, 10,8 bzw. 10,4 Angström.

11. Das Verfahren gemäß Anspruch 10, ferner umfassend das Isolieren des Form-V-Polymorphen.

12. Das Verfahren gemäß Anspruch 11, wobei das Isolieren des Form-V-Polymorphen durch Filtration erfolgt.

13. Das Verfahren gemäß Anspruch 10, wobei die erhöhte Temperatur 35-70°C beträgt.

14. Das Verfahren gemäß Anspruch 13, wobei die erhöhte Temperatur 50-65°C beträgt.

15. Das Verfahren gemäß Anspruch 10, wobei die niedrige Temperatur 0-30°C beträgt.

16. Das Verfahren gemäß Anspruch 15, wobei die niedrige Temperatur 10-20°C beträgt.

17. Das Verfahren gemäß Anspruch 10, wobei die erhöhte Temperatur 50-65°C und die niedrige Temperatur etwa 10-20°C beträgt.

## Revendications

1. Forme polymorphe du composé de la formule A: 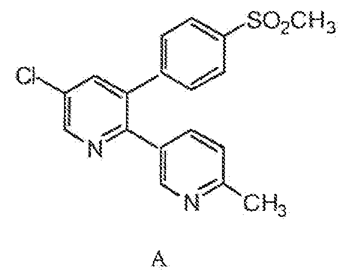 qui est désignée Forme V, **caractérisée** comme ayant une position de pic sur le réseau de diffraction des rayons X sur poudre à 13,7 angströms, Cu K alpha.

2. Forme polymorphe selon la revendication 1 **caractérisée en** outre comme ayant au moins une position de pic sur le réseau de diffraction des rayons X sur poudre à: 7,2, 6,9, 6,7, 5,8, 5,7, 5,0, 4,9, 4,8, 4,7, 4,5, 4,2, 4,0, 3,9, 3,8, 3,7, 3,6, 3,4, 3,3, 3,1, 3,0, 2,9, ou 2,8 angströms, Cu K alpha.

3. Forme polymorphe selon la revendication 1 **caractérisée** comme ayant un début de température de fusion extrapolé de 133°C à la DSC.

4. Forme polymorphe selon la revendication 1 **caractérisée** comme ayant un pic de température de fusion de 134,5°C à la DSC.

5. Forme polymorphe selon la revendication 1 sous forme sensiblement pure.

6. Composition pharmaceutique comprenant une quantité non toxique thérapeutiquement efficace d'une forme polymorphe selon la revendication 1 et un excipient pharmaceutiquement acceptable.

7. Forme polymorphe selon l'une quelconque des revendications 1 à 5 à utiliser dans une méthode de traitement du corps humain ou animal par thérapie.

8. Utilisation d'une forme polymorphe selon l'une quelconque des revendications 1 à 5 dans la fabrication d'un médicament pour traiter une maladie inflammatoire et/ou traiter une maladie médiée par la cyclo-oxygénase.

9. Forme polymorphe selon l'une quelconque des revendications 1 à 5 à utiliser dans le traitement d'une maladie sélectionnée parmi:
(a) fièvre rhumatismale,
(b) symptômes associés à la grippe ou autres infections virales, rhume commun,
(c) douleur dans le bas du dos et le cou,
(d) dysménorrhée,
(e) mal de tête,
(f) mal de dents,
(g) foulures et froissements,
(h) myosite,
(i) névralgie,
(j) synovite,
(k) arthrite, y compris polyarthrite rhumatoïde, maladies articulaires dégénératives (ostéoarthrite), goutte et spondylite ankylosante,
(l) bursite,
(m) brûlures,
(n) blessures, et
(o) à la suite de procédures chirurgicales et dentaires.

10. Procédé de fabrication d'un polymorphisme de Forme V de la formule développée A: 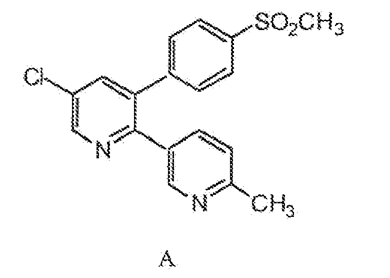 tel que défini dans la revendication 1, comprenant:
combiner un polymorphisme I, II, III ou IV ou un mélange quelconque de polymorphismes du Composé A avec de l'acétate d'isopropyle;
chauffer à une température élevée de moins d'environ 75°C; et
refroidir à une température basse pour produire le polymorphisme de Forme V;
où lesdits polymorphismes I, II, III et IV sont **caractérisés par** les positions des pics sur le réseau de diffraction des rayons X sur poudre à 12,6, 16,1, 10,8 et 10,4 angströms, Cu K alpha.

11. Procédé selon la revendication 10, comprenant en outre isoler le polymorphisme de Forme V.

12. Procédé selon la revendication 11, dans lequel l'isolement du polymorphisme de Forme V est fait pas filtration.

13. Procédé selon la revendication 10, dans lequel la température élevée est de 35-70°C.

14. Procédé selon la revendication 13, dans lequel la température élevée est de 50-65°C.

15. Procédé selon la revendication 10, dans lequel la température basse est de 0-30°C.

16. Procédé selon la revendication 15, dans lequel la température basse est de 10-20°C.

17. Procédé selon la revendication 10, dans lequel la température élevée est de 50-65°C et la température basse est d'environ 10-20°C.
